# EUROPÄISCHE PATENTSCHRIFT

(11) **EP 2 705 825 B1**
(45) Veröffentlichungstag und Bekanntmachung des Hinweises auf die Patenterteilung: **31.10.2018**
(21) Anmeldenummer: 12183620.9
(22) Anmeldetag: 07.09.2012
(51) Int. Cl.: A61K 6/00

(54) **Fluoridhaltiger Lack zum Auftrag auf die Zahnoberfläche**
Varnish containing fluoride for application to the surface of teeth
Laque contenant du fluorure à appliquer sur la surface des dents

(43) Veröffentlichungstag der Anmeldung: 12.03.2014
(73) Patentinhaber: Ivoclar Vivadent AG, 9494 Schaan (LI)
(72) Erfinder: Lendenmann, Urs, 9472 Grabs (CH); Bolis, Carlo, 7206 Igis (CH)
(74) Vertreter: Uexküll & Stolberg

(56) Entgegenhaltungen:
- US-A1- 2006 134 012
- US-A1- 2010 316 726
- US-A1- 2011 097 368
- US-B2- 7 101 942
- US-B2- 7 264 882

## Beschreibung

Die Erfindung betrifft einen fluoridhaltigen Lack zum Auftrag auf die Zahnoberfläche, der sich insbesondere zur Verwendung bei der Behandlung überempfindlicher Zähne und/oder Zahnhälse, bei der Kariesprophylaxe, bei der Behandlung beginnender Kariesläsionen, der Inhibierung von Demineralisation und/oder Zahnerosion eignet.

In den letzten Jahrzehnten haben Fluoride zu einem eindrücklichen Rückgang von Karieserkrankungen bei Kindern geführt. Während die kariesmindernde Wirkung von Fluoriden in permanenten Zähnen von Kindern klinisch deutlich nachgewiesen wurde, ist dieser Erfolg von Fluorid leider begleitet von häufigeren Befunden von Fluorose, besonders wenn Fluorid als Nahrungsergänzung oder über Trinkwasser verabreicht wird. Schon bei Fluoridkonzentrationen größer als 1 ppm im Trinkwasser kann Fluorose verstärkt auftreten. Bei Kindern unter 6 Jahren werden von der American Dental Association ausschließlich Fluoridlacke zur Fluoridierung empfohlen, um das Risiko einer Fluoridvergiftung durch Verschlucken gering zu halten.

Fluoridlacke für die gezielte, lokale Applikation zur Prophylaxe von Karies sind schon seit Jahren auf dem Markt. Die kariesprophylaktische Wirkung beruht auf der Fluoridierung des Zahns, genauer der Deposition einer calciumfluorid-ähnlichen Schicht auf dem Zahn sowie einer Erhöhung des Fluoridgehaltes in den obersten Schmelzschichten. Dadurch wird das Auflösen des Zahnschmelzes, welcher hauptsächlich aus Hydroxylapatit besteht, bei einem Säureangriff verlangsamt. Fluorid beschleunigt ebenfalls die Kristallisation von Hydroxylapatit. Bereits leicht demineralisierter Schmelz benötigt, verglichen mit gesundem Schmelz, im umgebenden Medium wesentlich höhere Fluoridkonzentration, um die weitere Demineralisation zu verhindern.

Aktuelle am Markt befindliche Fluoridlacke, welche ohne Lichtpolymerisation auskommen, sind hauptsächlich Suspensionen von Natriumfluorid in einer Lösung von Naturharzen.

Das Produkt Duraphat® (Colgate) enthält neben Natriumfluorid verschiedene Harze wie Kolophonium, Mastix und Schellack. Als Lösungsmittel dient Ethanol. Es wurde gezeigt, dass Duraphat® in der Prävention der Wurzelkaries, zur Behandlung hypersensibler Zahnhälse und zur Verlangsamung der Progressionsrate von erosiven Zahnveränderungen wirksam ist. Die Fluoridakkumulation ist von der Applikationszeit abhängig. Eine sechsstündige Applikation führte zu einer wesentlich höheren Fluoridanreicherung als eine einstündige Applikation (Hellwig, Oralprophylaxe & Kinderzahnheilkunde 30 (2008) 4, 139-143).

Clinpro® White Varnish (3M-ESPE) enthält 5 % Natriumfluorid und Tricalciumphosphat (TCP) in einer alkoholischen Lösung von modifizierten Harzen. Die Harze sind weiß oder zahnfarben, so dass das Produkt nach dem Auftrag praktisch nicht sichtbar ist. *In vitro* soll die Fluoridfreisetzung über einen Zeitraum von 24 Stunden anhalten.

MI Varnish® (GC Corp.) enthält neben 5 % Natriumfluorid hydriertes Kolophonium und CPP-ACP (Caseinphosphopeptid - amorphes Calciumphosphat) in einer Lösung von Polyvinylacetat in Ethylalkohol. Es versorgt die Zahnoberfläche mit bioverfügbarem Calcium, Phosphat und Fluorid.

Daneben werden Suspensionen von Natrium- und Calciumfluorid in einer Lösung von Nitrocellulose in einer Mischung aus Ethylacetat und Isopentylpropionat (Bifluorid 10, Bifluorid 12; VOCO) zur Kariesprävention und Behandlung von Hypersensibilität eingesetzt.

Das Produkt Fluor Protector® (Ivoclar-Vivadent) enthält eine Mischung eines Fluorsilans und eines Polyisocyanats gelöst in einer Mischung aus Ethylacetat und Isoamylpropionat.

Das Dokument US 2011/097368 beschreibt einen Fluoridhaltigen Lack zum Auftrag auf die Zahnoberfläche, in dem eine anorganische Fluoridquelle in nicht gelöster Form vorliegt. Die bekannten Produkte sind nicht in jeder Hinsicht zufriedenstellend. Suspendiertes Fluorid kann sich während der Lagerung absetzen, so dass Suspensionen vor der Applikation häufig durch Mischen homogenisiert werden müssen. Nach der Applikation muss sich das suspendierte Fluorid zudem erst lösen, damit es mit dem Hydroxylapatit des Zahns wechselwirken kann, was die Fluoridfreisetzung verzögert. Eine breite Korngrößenverteilung der Fluoridpartikel kann außerdem eine unregelmäßige Fluoridfreisetzung zur Folge haben.
Die Fluoridquelle Fluorsilan ist zwar in organischen Lösungsmitteln löslich, die anfängliche Fluoridfreisetzung wird jedoch dadurch verzögert, dass das Fluorsilan zuerst hydrolysiert werden muss.
Die Lacke werden meist mit einem kleinen Pinsel oder einem Schwämmchen auf die Zahnoberfläche aufgebracht. Dabei ist es von Vorteil, wenn ein dünner Film erzeugt werden kann, welcher nach der Behandlung vom Patienten als möglichst wenig störend empfunden wird. Bei vielen Suspensionen handelt es sich jedoch um hochviskose Flüssigkeiten, die relativ dicke Filme ergeben.
Die für eine ausreichende Fluoridierung nötigen hohen Natriumfluoridkonzentrationen erhöhen, speziell bei einer hohen Viskosität der Lacke und den daraus resultierenden dicken und damit viel Fluorid enthaltenden Lackfilmen, das Risiko einer Fluorose, da die sich mit der Zeit ablösenden Lackfilme häufig verschluckt werden.
Naturharze wie Kolophonium, Kolophoniumderivate, Mastix oder Schellack sind hellgelb bis bernsteinfarbig und bewirken eine starke Färbung der Lacke. Die Lackfilme sind daher nach dem Auftrag auf die Zahnoberfläche während der Tragedauer sichtbar, was von vielen Patienten als störend empfunden wird. Das suspendierte Natriumfluorid bewirkt zwar eine Aufhellung des Lackfilms, dies geschieht aber auf Kosten der Transparenz, d.h. die Suspension und auch der getrocknete Film wirken opak und heben sich von der Zahnoberfläche optisch ab. Kolophonium kann allergische Reaktionen und Asthma auslösen und Ekzeme verursachen.

Andere Lackkomponenten wie Polyisocyanate reagieren leicht mit Wasser zu Polyharnstoffen, was eine aufwendige, wasserdichte Verpackung für Mehrfachanwendungen erforderlich macht. Außerdem muss die Zahnoberfläche vor der Applikation gut getrocknet werden, was speziell bei Kindern nicht einfach zu erreichen ist.

Lösungsmittel wie Ethylacetat und Isoamylpropionat haben einen starken und vielfach als unangenehm empfundenen Geruch.

Der Erfindung liegt die Aufgabe zugrunde, einen fluoridhaltigen Lack für die Zahnbehandlung zur Verfügung zu stellen, der die oben beschriebenen Nachteile nicht aufweist. Insbesondere soll der Lack bei geringer Fluoridkonzentration eine wirksame Fluoridierung und Remineralisierung des Zahnschmelzes ermöglichen. Der Lack soll eine möglichst geringe Viskosität haben und die Herstellung farbloser Lackfilme erlauben.

Erfindungsgemäß wird diese Aufgabe durch fluoridhaltige Lacke gelöst, die

| | |
|---|---|
| 25 - 87,5 Gew.-% | organisches Lösungsmittel, |
| 2 - 50 Gew.-% | Wasser, |
| 5 - 50 Gew.-% | wasserunlöslichen Filmbildner, |
| 0,5 - 10 Gew.-% | anorganische Fluoridquelle in gelöster Form und |
| 5 - 25 Gew.-% | Weichmacher |

enthalten, jeweils bezogen auf die Gesamtmasse des Lacks. Bevorzugt sind Lacke, die
45 - 80 Gew.-%, insbesondere 48 - 70 Gew.-% organisches Lösungsmittel,
5 - 25 Gew.-%, insbesondere 10 - 20 Gew.-% Wasser,
5 - 25 Gew.-%, insbesondere 8 - 20 Gew.-% wasserunlöslichen Filmbildner,
5 - 22 Gew.-%, insbesondere 10 - 20 Gew.-% Weichmacher und
0,5 - 5 Gew.-%, insbesondere 1 - 3 Gew.-% anorganische Fluoridquelle in gelöster Form
enthalten.

Es wurde überraschend gefunden, dass die erfindungsgemäßen Lacke auch bei relativ geringen Fluoridmengen eine hohe Fluoridfreisetzung zeigen und insbesondere dass das freigesetzte Fluorid sehr effektiv vom Zahnschmelz aufgenommen wird. Produkte nach dem Stand der Technik, in denen die Fluoridquelle (meist Natriumfluorid) als suspendiertes Pulver vorliegt, müssen lange auf dem Zahn verbleiben, da sich das Fluorid zunächst lösen muss, bevor es vom Zahnschmelz aufgenommen werden kann. Das Auflösen des Fluorids geschieht durch Wasser aus dem Speichel. Da bekannte Lacke meist wasserfrei und hydrophob sind, kommt der Speichel nur oberflächlich mit der Lackschicht in Kontakt und löst Fluorid heraus. Problematisch ist dabei weiterhin, dass die freigesetzten Fluoridionen nur schwer durch den hydrophoben Lackfilm hindurch in Richtung Zahnoberfläche diffundieren können und daher zum größten Teil in die Mundhöhle abwandern. Das führt dazu, dass bekannte Produkte zwar große Mengen an Fluorid freisetzen können, aber nur wenig Fluorid auf den Schmelz gelangt.

Erfindungsgemäß bevorzugte Weichmacher zur Herstellung der Lacke sind Fettalkohole, Polyethylenglykol(PEG), Polypropylenglykol(PPG), Dexpanthenol und vorzugsweise Ester, wie Zuckerester und Alkyl- oder Phenylester von Di- oder Tricarbonsäuren oder Hydroxydi- oder tricarbonsäuren mit einer oder mehreren Hydroxylgruppen.

Bevorzugte Ester sind beispielsweise Alkyl- oder Phenylester (C₁₂ und höher) von Dicarbonsäuren, Hydroxydicarbonsäuren mit einer (vorzugsweise Diisostearyl Malate, erhältlich z.B. von der Firma Lubrizol unter der Bezeichnung Schercemol™ DISM Ester) oder mehreren Hydroxylgruppen; Alkyl- oder Phenylester von Polyethylenglykolen oder Polypropylenglykol oder Kombinationen davon (vorzugsweise PEG/PPG-8/3 Diisostearate, erhältlich z.B. von der Firma Lubrizol unter der Bezeichnung Hydramol™ PGPD Ester); Alkyl- oder Phenylester von Glycerin, Di- oder Triglycerin (vorzugsweise Polyglyceryl-3 Laurate, erhältlich z.B. von der Firmal Lubrizol unter der Bezeichnung Hydramol™ TGL Ester;); Alkyl- oder Phenylester von Di- und Tricarbonsäuren oder Hydroxydi- und Tricarbonsäuren mit Guerbetalkoholen (C₁₆ und höher), vorzugsweise Trioctyldodecylcitrat, Adipinsäurepolyester (erhältlich z.B. von der Firma Lanxess unter der Bezeichnung Ultramoll®), Alkylsulfonsäurephenylester (erhältlich z.B. von der Firma Lanxess unter der Bezeichnung Mesamoll®), Ester von hydriertem Kolophonium, Fettalkohole, Dexpanthenol, Polyethylenglykol (PEG), Polypropylenglykol (PPG).

Als Weichmacher eignen sich besonders Zuckerester, insbesondere Ester einer organischen Säure, vorzugsweise einer Monocarbonsäure mit 1 bis 18, insbesondere 1 bis 4 Kohlenstoffatomen, mit einem Mono- oder Disaccharid, wobei solche Ester besonders bevorzugt sind, in denen die Hydroxylgruppen der Zuckerkomponente vollständig verestert sind. Ganz besonders bevorzugt sind Ester von Saccharose mit Essigsäure oder Isobuttersäure oder gemischte Ester von Saccharose mit Essigsäure und Isobuttersäure und insbesondere Saccharoseacetatisobutyrat (SAIB; INCI-Name: sucrose acetate isobutyrate). SAIB ist eine Mischung unterschiedlicher Isomere, deren Zusammensetzung in etwa Saccharosediacetathexaisobutyrat entspricht. Es handelt sich um eine hochviskose Flüssigkeit. SAIB ist ein Emulgator und Lebensmittelzusatzstoff (E-Nummer 444).

Die erfindungsgemäßen Lacke zeichnen sich dadurch aus, dass sie Wasser enthalten. Ganz besonders bevorzugt sind Lacke, die eine Mischung von Wasser und einem mit Wasser mischbaren organischen Lösungsmittel enthalten. Bevorzugte wassermischbare organische Lösungsmittel sind ein- oder mehrwertige C₂-C₄-Alkohole oder C₂-C₄-Ketone und Mischungen davon, besonders bevorzugt Isopropanol, Aceton und ganz besonders bevorzugt Ethanol.

Erfindungsgemäß bevorzugte Lacke enthalten Ethanol, wobei das Verhältnis von Ethanol und Wasser so bemessen ist, dass die Mischung 10 bis 30 Gew.-% Wasser und 70 bis 90 Gew.-% Ethanol enthält, jeweils bezogen auf die Gesamtmenge an Ethanol und Wasser.

Gemäß einer Ausführungsform enthalten die erfindungsgemäßen Lacke vorzugsweise keine unangenehm riechenden Lösungsmittel wie z.B. Ethylacetat, Isoamylpropionat, Isopentylpropionat und Alkane, wie C₅-C₁₂-Alkane, beispielsweise n-Hexan.

Als Fluoridquelle werden vorzugsweise anorganische Fluoride, Bifluoride (Hydrogendifluoride) oder Fluorkomplexsalze verwendet, besonders bevorzugt NH₄F, KF, RbF, CsF, NH₄HF₂, KHF₂, Natriummhexafluorophosphat, ganz besonders bevorzugt NH₄F und KF. Als Fluoridquelle kann auch eine Mischung von zwei oder mehr dieser Stoffe eingesetzt werden. Die Fluoridquelle wird im Folgenden der Einfachheit halber auch als Fluorid bezeichnet.

Die Fluoridquelle soll gut in Wasser löslich sein, d.h. eine Löslichkeit bei 25°C von mindestens 5 mol in 1 kg Wasser, bevorzugt mindestens 10 mol in 1 kg Wasser aufweisen. Erfindungsgemäß besonders geeignete Fluoride und deren Löslichkeiten sind wie folgt:

| **Fluoridquelle** | **Löslichkeit (g/100 g H₂O)** | **Löslichkeit (mol/kg H₂O)** |
|---|---|---|
| Kaliumbifluorid; KHF₂ | 39,2 | 5,0 |
| Natriumhexafluorophosphat monohydrat; NaPF₆ x H₂O | 103 | 5,5 |
| Ammoniumbifluorid; NH₄HF₂ | 60,2 | 10,6 |
| Silber(I)fluorid; AgF | 172 | 13,6 |
| Kaliumfluorid; KF | 102 | 17,6 |
| Ammoniumfluorid; NH₄F | 83,5 | 22,5 |
| Rubidiumfluorid; RbF | 300 | 28,7 |
| Cäsiumfluorid; CsF | 573 | 37,7 |

Die erfindungsgemäße Lacke haben den Vorteil, dass die Fluoridquelle bereits im Lack in gelöster Form vorliegt, so dass es zu keinen Verzögerungen der Fluoridfreisetzung durch Auflösevorgänge kommt. Die Menge an gelöstem Fluorid lässt sich durch die Art der Fluoridquelle, Art und Menge des Lösungsmittels und den Wassergehalt des Lacks steuern.

Die erfindungsgemäßen fluoridhaltigen Lacke enthalten als Filmbildner vorzugsweise ein alkohol- oder ketonlösliches, vorzugsweise ein ethanollösliches Polymer. Der Begriff alkohol- oder ketonlöslich bezieht sich auf die oben genannten Lösungsmittel. Der Filmbildner ist in Wasser unlöslich. Bevorzugt sind Polymere, die in Ethanol oder Mischungen aus Ethanol und bis zu 50 Gew.-% Wasser eine Löslichkeit von mindestens 6 Gew.-% und vorzugsweise mindestens 10 Gew.-% aufweisen. Unter wasserunlöslichen Polymeren werden solche Substanzen verstanden, die in Wasser eine Löslichkeit von maximal 5 Gew.-%, insbesondere maximal 4 Gew.-% aufweisen. Wenn nicht anders angegeben beziehen sich alle Löslichkeitsangaben hierin auf eine Temperatur von 25 °C.

Als Filmbildner eignen sich neutrale, kationische und insbesondere anionische filmbildende Polymere.

Beispiele für anionische Polymere sind Homo- und Copolymerisate von Acrylsäure und Methacrylsäure, Copolymere von Acrylsäure, Acrylaten und Acrylamid, sowie Homo- und Copolymerisate von Acrylsäure- und Methacrylsäureestern.

Besonders geeignete Polymere sind Copolymere aus t-Butylacrylat, Ethylacrylat, Methacrylsäure (z.B. Luvimer® 100P), Copolymere aus Ethylacrylat und Methacrylsäure (z.B. Luviflex® Soft und Luvimer® MAE), Copolymere aus N-tert.-Butylacrylamid, Ethylacrylat, Acrylsäure (Ultrahold® 8 strong der BASF AG), Copolymere aus Vinylacetat, Crotonsäure und gegebenenfalls weitere Vinylester (z.B. Luviset®, BASF AG), anionische Polysiloxane, z.B. carboxyfunktionelle, t-Butylacrylat, Methacrylsäure (z.B. Luviskol® VBM, BASF AG). Beispiele für anionische Polymere sind weiterhin Vinylacetat/Crotonsäure-Copolymere, wie sie beispielsweise unter den Bezeichnungen Resyn® (Akzo Nobel) und Gafset® (GAF) im Handel sind.

Weiterhin umfasst die Gruppe der erfindungsgemäß als Filmbildner geeigneten Polymere Balance® CR (Akzo Nobel; Acrylate Copolymer), Balance® 0/55 (Akzo Nobel; Acrylate Copolymer), Balance® 47 (Akzo Nobel; Octylacrylamid/Acrylate/Butylaminoethylmethacrylate Copolymer), Amphomer® HC (Akzo Nobel; Acrylat/Octylacrylamid Copolymer), Amphomer® 28-4910 (Akzo Nobel; Octylacrylamid/Acrylat/Butylaminoethylmethacrylat Copolymer), Advantage® Plus (lSP; VA/Butyl Maleate/lsobornyl Acrylate Copolymer), Luviflex® Silk (BASF; Reaction product of t-butyl acrylate, methacrylic acid and dimethicone copolyol), Resyn XP (Akzo Nobel; Acrylates/Octylacrylamide Copolymer).
Geeignete Polymere sind auch amphotere Polymere, wie die unter der Bezeichnung Amphomer® (Akzo Nobel) erhältlichen Octylacrylamid/Methylmethacrylat/tert. Butylaminoethylmethacrylat/2-Hydroxypropylmethacrylat Copolymere.
Besonders bevorzugte anionische Filmbildner sind Acrylat- und Methacrylathomopolymere, Acrylat- und Methacrylatcopolymere, Terpolymere aus C₄-C₈-Alkyl-Acrylamid, Acrylaten und Acrylsäure, Terpolymere aus Methacrylsäure und Acrylaten, Vinylacetat-Crotonsäure-Copolymere und Mischungen davon. Ganz besonders bevorzugte Filmbildner sind Terpolymere von tert-Butylacrylat, Ethylacrylat und Methacrylsäure (INCI-Name: acrylates copolymer).
Die Polymere werden vorzugsweise in ionischer und nicht in neutralisierter Form eingesetzt.
Es hat sich überraschend gezeigt, dass mit den erfindungsgemäßen Lacken die einen Weichmacher enthalten, bereits nach kurzer Zeit ein hoher Fluoridgehalt auf dem Zahnschmelz erzielt wird. Es wird angenommen, dass nach dem Trocknen der Lacke unter intraoralen Bedingungen genügend Wasser im Film verbleibt, um das gelöstes Fluorid ganz oder teilweise in Lösung zu halten. Das in den Lacken enthaltende organische Lösungsmittel verdampft beim Trocknen schneller als der Wasseranteil, so dass trotz des Aufkonzentrierens die Fluoridquelle auch nach dem Trocknen zumindest noch teilweise in gelöster Form vorliegt. Der Weichmacher wirkt sich dabei positiv auf den Restwassergehalt des Films aus. Das verbleibende Wasser im Lackfilm erlaubt eine schnelle Diffusion der Fluoridionen in Richtung Zahnschmelz, so dass schnell ein hoher Fluoridgehalt auf dem Zahnschmelz erzielt wird.

Der Weichmacher verhindert bei der Aushärtung des Lacks auf den Zähnen außerdem, dass der Filmbildner inhomogen austrocknet oder präzipitiert und dass sich zu große kristalline Domänen des Filmbildners ausbilden.

Besonders vorteilhaft ist, dass die erfindungsgemäßen Lacke nach topischer Applikation auf den Zahnschmelz schon nach kurzer Zeit zu einer deutlichen Erhöhung der Fluoridkonzentration auf und im Schmelz führen. Für oberflächliches, alkalilösliches Fluorid (ASF; "auf Schmelz") werden nach einer Stunde Behandlungsdauer Werte von 10 bis 50 µg/cm² erzielt, und für strukturell gebundenes Fluorid (SBF; "im Schmelz") Werte von 5 bis 20 µg/cm². Strukturell gebundenes Fluorid ist Fluorid, das in die Hydroxylapatit-Kristalle des Zahnschmelzes eingebaut wird und kann nach Ätzen der Schmelzoberfläche mit HClO₄ bestimmt werden. Die Bestimmung des Fluorids auf dem Schmelz und im Schmelz wird in Beispiel 2 beschrieben. Die schnelle Erhöhung der Fluoridkonzentration auf und im Schmelz ermöglicht eine Behandlungszeit von 1 Stunde oder weniger, vorzugsweise 0,5 bis 1 Stunde.

Bevorzugt sind Lacke, die nach einer Stunde Behandlungsdauer eine Schmelzfluoridierung (ASF) von mindestens 10 µg/cm², insbesondere 10 bis 50 µg/cm², bevorzugt mindestens 15 µg/cm², insbesondere 15 bis 50 µg/cm², ergeben, gemessen an Rinderzahnschmelz analog zu Beispiel 2.

Neben den genannten Komponenten können die erfindungsgemäßen Lacke übliche Additive wie Aromastoffe, Süßungsmittel, Füller und antibakterielle Wirkstoffe enthalten. Diese Stoffe werden üblicherweise in Mengen von jeweils 0,01 bis 10 Gew.-%, bevorzugt 0,01 bis 5 Gew.-%, besonders bevorzugt 0,01 bis 2 Gew.-% eingesetzt.

Als Aromastoffe eignen sich natürliche und künstliche Aromen oder Aromaextrakte mit Geruch / Geschmack nach Pfefferminz, Orange, Erdbeere, Vanille, Melone etc. Geeignete Aromen werden in "ENTSCHEIDUNG DER KOMMISSION vom 23. Februar 1999 über ein Verzeichnis der in oder auf Lebensmitteln verwendeten Aromastoffe" aufgeführt, das gemäß Verordnung (EG) Nr. 2232/96 des Europäischen Parlaments und des Rates vom 28. Oktober 1996 erstellt wurde(1999/217/EG).

Als Süßungsmittel eignen sich besonders natürliche und synthetische Zuckerersatzstoffe wie Zuckeralkohole (Sorbit, Mannit, Xylit etc.) und Süßstoffe (Saccharin, Acesulfam, Aspartam, Cyclamat, Neohesperidin, Sucralose, Steviosid etc.).

Als Füller eignen sich besonders gefällte oder pyrogene Kieselsäuren (z.B. Aerosil der Firma Evonik), nanoskalige Metalloxide wie z.B. pyrogenes Aluminiumoxid (z.B. Aeroxid Alu C, Evonik) oder mineralische Füller wie z.B. Schichtsilikate.

Als antibakterielle Wirkstoffe eignen sich besonders Peroxide wie Wasserstoffperoxid oder Carbamidperoxid, des weiteren Chlorhexidin, Cetylpyridiniumchlorid, Hydroxybenzoesäureester (Parabene) etc.

Lacke dieser Erfindung können auch weitere anorganische Stoffe enthalten, beispielsweise Mineralquellen, die die Remineralisation von Zähnen fördern. Beispiele sind Amorphes Calciumphosphat, Tricalciumphosphat, Calciumcarbonat, Hydroxylapatit, Fluorapatit, Kaliumphosphat etc. Diese Stoffe liegen idealerweise so fein vor, dass sie in einer niedrigviskosen Flüssigkeit suspendiert bleiben. Darum sind fein gemahlene oder nanoskalige Teilchengrößen solcher Stoffe gewünscht. Bevorzugt sind Partikelgrößen von 0,005 µm bis 10 µm, besonders bevorzugt 0,005 µm bis 1 µm und ganz besonders bevorzugt 5 nm bis 200 nm.

Besonders vorteilhaft sind naturgemäß solche Lacke, die eine Kombination von bevorzugten und insbesondere von besonders bevorzugten Komponenten enthalten oder daraus bestehen. Solche Kombinationen sind daher bevorzugt.
Eine ganz besonders vorteilhafte Kombination ist beispielsweise ein Lack, der

| | |
|---|---|
| 50 - 70 Gew.-% | Ethanol, |
| 10 - 20 Gew.-% | Wasser, |
| 5 - 15 Gew.-% | Terpolymer von tert-Butylacrylat, Ethylacrylat und Methacrylsäure (INCI: Acrylates copolymer), |
| 10 - 20 Gew.-% | Saccharoseacetatisobutyrat, |
| 1 - 2 Gew.-% | Ammoniumfluorid |
| 0,05 - 0,2 Gew.-% | Aromastoff(e) und |
| 0,01 bis 0,1 Gew.-% | Süßungsmittel enthält. |

Ein Beispiel von Fluoridlack ohne Weichmacher ist:

| | |
|---|---|
| 60 - 75 Gew.-% | Ethanol |
| 7,5 - 20 Gew.-% | Wasser |
| 10 - 25 Gew.-% | Terpolymer von tert-Butylacrylat, Ethylacrylat und Methacrylsäure (INCI: Acrylates copolymer) |
| 0,01 - 0,1 Gew.-% | Süssungsmittel |
| 1 - 2 Gew.-% | Ammoniumfluorid |
| 0,1 - 0,5 Gew.-% | pyrogenes Aluminiumoxid |
| 0,05 - 0,2 Gew.-% | Aromastoff(e). |

Die erfindungsgemäße Lacke können mit einem Pinsel oder Schwämmchen schnell auf den Zahn aufgetragen werden und bilden dort nach Verdunsten der Lösungsmittel einen farblosen, klaren und gut haftenden Lackfilm, der eine schnelle und hohe Fluoridfreisetzung aufweist und schon nach kurzer Applikationszeit von einer Stunde eine große Menge alkalilösliches Fluorid auf der Schmelzoberfläche ergibt. Die Lacke ergeben eine calciumfluoridähnliche Schicht auf dem Schmelz und reichern den Schmelz unterhalb der Oberfläche mit Fluorid an. Es wird erstaunlicherweise eine mit höher konzentrierten Produkten mindestens gleichwertige Fluoridierung der obersten Schmelzschicht erzeugt.

Die erfindungsgemäßen Lacke zeichnen sich durch eine Kombination von vorteilhaften Eigenschaften aus. Sie weisen eine hohe initiale Fluoridfreisetzung auf und bilden nach dem Trocknen klare und gut haftende Lackfilme. Außerdem haben die Lacke eine sehr geringe Viskosität. Die Viskosität ist 10 bis 5.000 mPa·s, bevorzugt 10 bis 1.000 mPa·s und besonders bevorzugt 10 bis 250 mPa·s (gemessen mit einem Kegel-Platte-Messsystem bei 15,0°C und einer Scherrate von 100 s⁻¹).

Die erfindungsgemäßen Lacke eignen sich insbesondere zur Verwendung bei der Behandlung überempfindlicher Zähne und/oder Zahnhälse, bei der Kariesprophylaxe, bei der Behandlung beginnender Kariesläsionen, der Inhibierung von Demineralisation von Zähnen und/oder Zahnerosion, zum Schutz von Fissuren und Grübchen und zur Schmelzfluoridierung.

Im folgenden wird die Erfindung anhand von Figuren und Beispielen näher erläutert.

### Ausführungsbeispiele

### Beispiel 1

### Herstellung eines fluoridhaltigen Lacks

Durch Mischen der Komponenten wurde ein Lack mit der folgenden Zusammensetzung hergestellt:

| **Anteil** | **Komponente** |
|---|---|
| 58,70 % | Ethylalkohol puriss. |
| 14,68 % | Wasser entionisiert |
| 15,00 % | Saccharose acetat isobutyrat (SAIB) |
| 10,00 % | Acrylat Copolymer (Luvimer 100P, Fa. BASF) |
| 1,50 % | Ammoniumfluorid |
| 0,10 % | synth. Pfefferminzaroma |
| 0,02 % | Süßungsmittel (Saccharin) |

Sämtliche Bestandteile wurden unter Rühren im vorgelegten Lösungsmittel aufgelöst. Es wurde ein stabiler, praktisch farbloser, homogener, niedrigviskoser Lack erhalten.

### Beispiel 2

### In-vitro Schmelzfluoridierung durch Fluoridlacke (24h)

Zur Bestimmung der *in-vitro* Fluoridierung wurden aus Rinderzähnen zylindrische Schmelzblöcke (n=18) herausgebohrt und poliert (SiC, 4000 Grit). Danach wurden die Schmelzproben bei Raumtemperatur 1 Stunde mit Milchsäure demineralisiert (Demineralisationslösung in Anlehnung an Zahradnik et al, J Dent Res, 1976, 55; 4; 664-670, jedoch mit einer geringeren Calciumkonzentration wie in Speichel: Milchsäure 0.1 mol/1; Ca₃(PO₄)₂ 50 mg/l; NaN₃ 0.02 % (Konservierungsmittel); mit 2-5 M KOH auf pH 4.4 einstellen), dann gemäß Herstelleranweisungen mit den zu prüfenden Lacken beschichtet und getrocknet (25°C, 1h). Der erfindungsgemäße Lack wurde mit einem kleinen Pinsel (Microbrush®) auf den Schmelz aufgetragen und getrocknet (25°C, 1h). Die getrockneten Proben wurden in künstlichem Speichel gelagert (37°C, 1h bzw. 24h). Der künstliche Speichel wurde nach einer Stunde ausgetauscht, um die Bildung höherer Fluoridkonzentrationen im Elutionsmedium zu vermeiden. Anschließend wurden die Lacke mit Ethanol oder Aceton entfernt. Oberflächliches, alkali-lösliches Fluorid (ASF) und strukturell gebundenes Fluorid (SBF) wurden extrahiert und mit einer ionenselektiven Elektrode gemessen. Zur statistischen Analyse wurde ein ungepaarter t-Test durchgeführt, p<0,05.

Künstlicher Speichel wurde gemäß Takagi, Caries Res 1992; 26; 321-327 hergestellt, jedoch ohne Fluorid. Zusammensetzung: 1,2 mM CaCl₂ 2 H₂O, 0,72 mM KH₂PO₄, 30 mM KCl, 50 mM HEPES (= 4-(2-Hydroxyethyl)-1-Piperazinethanesulfonic Acid), pH 7.

Die Bestimmung des oberflächlichen, alkalilöslichen Fluorids (ASF) erfolgte gemäß Caslavska et al., Archs oral Biol 20; 333-339, 1975. Hierzu wurde das oberflächliche, CaF₂-ähnliche Fluorid durch 24-stündiges Eintauchen der Probe in 1M KOH gelöst, die Lösung dann mit HNO₃ neutralisiert und die Fluoridkonzentration nach Zugabe eines Puffers zur Einstellung der Gesamtionenstärke (TISAB II-Puffer, Herstellung: 58 g NaCl in 500 g deion. H₂O im 1.000 ml Becherglas lösen, 57 ml Eisessig und 4 g CDTA (1,2-Cyclohexylendiamintetraessigsäure) unter Rühren zufügen und lösen; danach mit 5 M NaOH einen pH-Wert von 5 - 5,5 einstellen (Kontrolle mit pH-Elektrode), Lösung abkühlen lassen und auf 1.000 ml mit deion. H₂O auffüllen) mit einer Fluoridelektrode gemessen.

Zur Bestimmung des strukturell gebundenen Fluorids (SBF) wurde eine Biopsie gemäß Sieck et al., J Dent Res 69; 1261-1265, 1990 durchgeführt. Die Oberfläche der Schmelzproben wurde für 1 Stunde mit 0,5M HClO₄ geätzt. Dabei wurden etwa 100 µm der Oberflächenschicht gelöst. Nach dem Neutralisieren und der Zugabe von TISAB-II wurde die Fluoridkonzentration wieder mit Fluoridelektrode gemessen. Die Ergebnisse sind in Tabelle 1 gezeigt.

**Tabelle 1: Ergebnisse der in vitro Schmelzfluoridierung nach 24h Behandlung / Lagerung in künstlichem Speichel**

| **Produkt** | **Hersteller** | **Fluoridkonz. [ppm]** | **ASF [µg/cm²]** | **SBF [µg/cm²]** |
|---|---|---|---|---|
| Beispiel 1 | | 7.700 | 20,4±6,2^{a} | 17,9±3,6^{c} |
| Duraphat*) | Colgate | 22.600 | 19,3±7,6^{a} | 21,0±6,9^{a} |
| Clinpro White Varnish*) | 3M-ESPE | 22.600 | 9,3±2,4^{b} | 11,5±1,8^{b} |
| Profluorid Varnish*) | VOCO | 22.600 | 11,6±2,9^{c} | 11,9±2,7^{b} |
| Kontrolle (Wasser) | | <1 | 1,4±0,8^{d} | 4,2±2,5^{d} |

| | | | | |
|---|---|---|---|---|
| *) Vergleichsbeispiel Werte mit gleichen hochgestellten Buchstaben unterscheiden sich nicht signifikant | | | | |

Nach dem Auftrag der Lacke auf den Zahnschmelz waren ASF und SBF in allen Proben im Vergleich zu Wasser signifikant erhöht.

Der erfindungsgemäße Lack erzielte nach topischer Applikation trotz deutlich geringerem Fluoridgehalt Spitzenwerte bei der Fluoridkonzentration auf (ASF) und im Schmelz (SPF).

### Beispiel 3

### EDX-Untersuchung der Schmelzfluoridierung

Die Fluoridierung von Schmelzoberflächen durch verschiedene Fluoridlacke wurde durch energiedispersive Röntgenanalyse (EDX) untersucht. Die untersuchten Lacke sind in Tabelle 2 gezeigt.

Zur Bestimmung der Schmelzfluoridierung wurden aus geschliffenen und polierten (SiC, 4.000 Grit) Rinderzähnen zylindrische Prüfkörper mit einem Durchmesser von 4mm ausgebohrt und demineralisiert (1h in 0,1M Milchsäure, eingestellt auf pH 4, 4) .

**Tabelle 2: Untersuchte Fluoridlacke**

| **Produkt** | **Batch** | **Hersteller** | **Fluoridquelle** | **Konz. Fluorid [ppm]** |
|---|---|---|---|---|
| Beispiel 1 | | | NH₄F | 7.700 |
| FluorProtector*) | PL1004 | Ivoclar-Vivadent | Fluorsilan | 1.000 |
| Duraphat*) | 116416 | Colgate | NaF | 22.600 |
| MI Varnish*) | 1110211 | GC Corp. | NaF | 22.600 |
| Clinpro White Varnish*) | M14300H1C | 3M-ESPE | NaF | 22.600 |
| Profluorid Varnish*) | 1114277 | VOCO | NaF | 22.600 |

| | | | | |
|---|---|---|---|---|
| *) Vergleichsbeispiel | | | | |

Die demineralisierten Schmelzoberflächen wurden mit Ausnahme der Negativkontrolle mit dem entsprechenden Fluoridlack behandelt (5 Min. antrockenen lassen) und danach für eine Stunde bei 37°C in künstlichem Speichel gelagert. Nach Herausnahme aus dem künstlichen Speichel wurde der Lack durch Schwenken des Prüfkörpers in reinem Ethanol (Beispiel 1) oder Aceton (restliche Lacke) entfernt und kurz mit Wasser nachgespült. Nach Trocknen der Prüfkörper mit Pressluft wurde die Schmelzoberfläche mittels EDX (Spotgrösse ca. 100 pm) untersucht. Die Ergebnisse sind in Tabelle 3 zusammengefasst.

Die Untersuchungsresultate zeigen, dass mit EDX unterschiedliche Fluoridgehalte im oberflächlichen Schmelz gemessen werden können. Dabei weist die mit Lack aus Beispiel 1 behandelte Probe mit Abstand den höchsten Fluorgehalt auf. Die vorliegende Untersuchung bestätigt die sehr gute Fluoridierungswirkung des Lacks aus Beispiel 1 auf demineralisierten Schmelz bereits nach kurzer Einwirkzeit.

**Tabelle 3: Resultate der EDX-Messungen**

| **Produkt** | **C** | **F** | **Na₂O** | **MgO** | **P₂O₅** | **Cl** | **K₂O** | **CaO** |
|---|---|---|---|---|---|---|---|---|
| Beispiel 1 | 9.27 | **4.15** | 0.89 | 0.31 | 36.49 | 0.00 | 0.56 | 48.32 |
| FluorProtector*) | 10.36 | **0.35** | 0.58 | 0.09 | 39.79 | 0.20 | 0.00 | 48.64 |
| Duraphat*) | 6.59 | **2.39** | 1.70 | 0.28 | 40.04 | 0.00 | 0.00 | 49.00 |
| MI Varnish*) | 5.69 | **0.57** | 1.33 | 0.31 | 41.02 | 0.00 | 0.00 | 51.08 |
| Clinpro White Varnish*) | 5.20 | **0.24** | 0.93 | 0.26 | 41.99 | 0.00 | 0.00 | 51.38 |
| Profluorid Varnish*) | 5.94 | **0.19** | 0.77 | 0.16 | 41.46 | 0.00 | 0.00 | 51.47 |
| Schmelz unbehandelt*) | 6.07 | **0.15** | 0.86 | 0.14 | 41.99 | 0.00 | 0.00 | 50.77 |

| | | | | | | | | |
|---|---|---|---|---|---|---|---|---|
| *) Vergleichsbeispiel | | | | | | | | |

### Beispiel 4

### In-vitro Schmelzfluoridierung durch Fluoridlacke (1h)

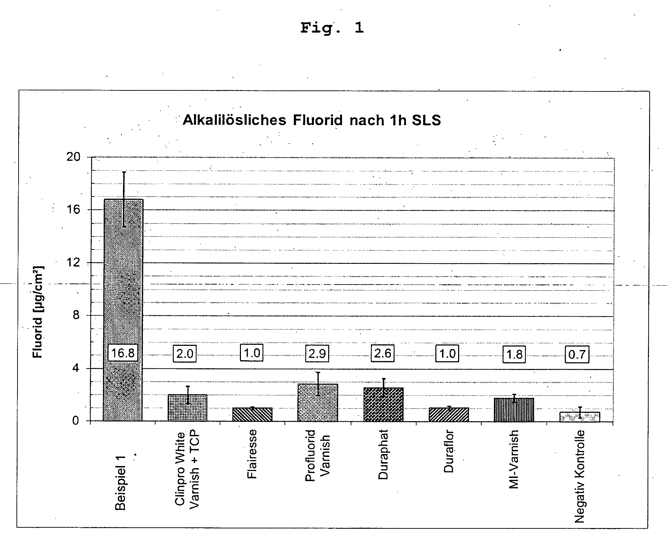

Analog zu Beispiel 2 wurden Schmelzblöcke aus Rinderzähnen mit fluoridhaltigen Lacken behandelt und das alkali-lösliche Fluorid (ASF) gemessen. Die getrockneten Proben wurden nur 1 Stunde in künstlichem Speichel (saliva-like solution, Abk.:SLS) bei 37°C gelagert. Die Ergebnisse sind in Tabelle 4 gezeigt und in Fig. 1 graphisch dargestellt.

**Tabelle 4: Ergebnisse der in vitro Schmelzfluoridierung nach 1h Behandlung**

| **Produkt** | **Hersteller** | **ASF [µg/cm²]** |
|---|---|---|
| Beispiel 1 | | 16,80±2,06 |
| Clinpro White Varnish + TCP*) | (3M-ESPE, Seefeld, DE) | 2,00±0,66 |
| Flairesse*) | (DMG, Hamburg, DE) | 1,00±0,11 |
| Profluorid Varnish*) | (VOCO, Cuxhaven, DE) | 2,86±0,89 |
| Duraphat*) | (Colgate, USA) | 2,58±0,68 |
| Duraflor*) | (Medicom, Lachine, CAN) | 1,03±0,14 |
| MI-Vamish *) | (GC Corp., Tokyo, J) | 1,78±0,32 |
| Negativ Kontrolle | | 0,72±0,42 |

| | | |
|---|---|---|
| *) Vergleichsbeispiel | | |

### Beispiel 5

### Herstellung eines fluoridhaltigen Lacks ohne Weichmacher

Durch Mischen der Komponenten wurde ein Lack mit der folgenden Zusammensetzung hergestellt:

| **Anteil** | **Komponente** |
|---|---|
| 57,90 Gew.-% | Ethanol |
| 14,48 Gew.-% | Wasser |
| 25,00 Gew.-% | Terpolymer von tert-Butylacrylat, Ethylacrylat und Methacrylsäure (INCI: Acrylates copolymer) |
| 0,02 Gew.-% | Saccharin-Natrium |
| 2,0 Gew.-% | Ammoniumfluorid |
| 0,50 Gew.-% | pyrogenes Aluminiumoxid |
| 0,10 Gew.-% | Pfefferminzaroma |

Sämtliche Bestandteile wurden unter Rühren im vorgelegten Lösungsmittel aufgelöst. Es wurde ein stabiler, praktisch farbloser, homogener, niedrigviskoser Lack erhalten.

## Patentansprüche

1. Fluoridhaltiger Lack zum Auftrag auf die Zahnoberfläche, **dadurch gekennzeichnet, dass** er
| | |
|---|---|
| 25 - 87,5 Gew.-% | organisches Lösungsmittel, |
| 2 - 50 Gew.-% | Wasser, |
| 5 - 50 Gew.-% | wasserunlöslichen Filmbildner, |
| 0,5 - 10 Gew.-% | anorganische Fluoridquelle in gelöster Form und |
| 5 - 25 Gew.-% | Weichmacher |
enthält, jeweils bezogen auf die Gesamtmasse des Lacks.

2. Fluoridhaltiger Lack nach Anspruch 1, der als Weichmacher Fettalkohol, Polyethylenglykol (PEG), Polypropylenglykol (PPG), Dexpanthenol, einen Ester oder eine Mischung davon enthält.

3. Fluoridhaltiger Lack nach Anspruch 2, der als Weichmacher einen Zuckerester oder einen Alkyl- oder Phenylester von Di- oder Tricarbonsäuren oder Hydroxydi- oder tricarbonsäuren mit einer oder mehreren Hydroxylgruppen enthält.

4. Fluoridhaltiger Lack nach Anspruch 3, der als Zuckerester einen Ester einer organischen Säure mit einem Mono- oder Disaccharid enthält.

5. Fluoridhaltiger Lack nach Anspruch 4, der als Zuckerester einen Ester von Saccharose mit Essigsäure oder Isobuttersäure oder einen gemischten Ester von Saccharose mit Essigsäure und Isobuttersäure, insbesondere Saccharoseacetatisobutyrat enthält.

6. Fluoridhaltiger Lack nach einem der vorhergehenden Ansprüche, der als organisches Lösungsmittel einen mit Wasser mischbaren ein- oder mehrwertigen Alkohol und/oder ein mit Wasser mischbares Keton enthält, insbesondere Isopropanol, Aceton, ganz besonders bevorzugt Ethanol.

7. Fluoridhaltiger Lack nach einem der vorhergehenden Ansprüche, der eine anorganische Fluoridquelle enthält, die in Wasser eine Löslichkeit bei 25°C von mindestens 5 mol in 1 kg Wasser, bevorzugt mindestens 10 mol in 1 kg Wasser aufweist.

8. Fluoridhaltiger Lack nach Anspruch 7, der als anorganische Fluoridquelle NH₄F, KF, RbF, CsF, NH₄HF₂, KHF₂ oder eine Mischung davon enthält.

9. Fluoridhaltiger Lack nach einem der Ansprüche 1 bis 8, der als Filmbildner ein alkohol- oder ketonlösliches Polymer mit einer Wasserlöslichkeit von maximal 5,0 Gew.-% enthält.

10. Fluoridhaltiger Lack nach Anspruch 9, der als Filmbildner ein Acrylat- und Methacrylathomopolymer, Acrylat- und Methacrylatcopolymer, ein Terpolymer aus C₄-C₈-Alkyl-Acrylamid, Acrylaten und Acrylsäure, ein Terpolymer aus Methacrylsäure und Acrylaten, ein Vinylacetat-Crotonsäure-Copolymer, ein Terpolymer von tert-Butylacrylat, Ethylacrylat und Methacrylsäure (INCI-Name: acrylates copolymer) oder eine Mischung davon enthält.

11. Fluoridhaltiger Lack nach einem der vorhergehenden Ansprüche, der
50 - 70 Gew.-% Ethanol,
10 - 20 Gew.-% Wasser,
5 - 15 Gew.-% Terpolymer von tert-Butylacrylat, Ethylacrylat und Methacrylsäure,
10 - 20 Gew.-% Saccharoseacetatisobutyrat,
1 - 2 Gew.-% Ammoniumfluorid
0,05 - 0,2 Gew.-% Aromastoff(e) und
0,01 bis 0,1 Gew.-% Süßungsmittel
enthält.

12. Fluoridhaltiger Lack nach einem der Ansprüche 1 bis 11, der zusätzlich 0,01 - 10 Gew.-% Füller und/oder antibakterielle Wirkstoffe enthält.

13. Fluoridhaltiger Lack nach einem der Ansprüche 1 bis 12, der eine Viskosität von weniger als 5.000 mPa·s, bevorzugt weniger als 1.000 mPa·s und besonders bevorzugt weniger als 250 mPa s, gemessen bei 15,0°C und einer Scherrate von 100 s⁻¹, aufweist.

14. Fluoridhaltiger Lack nach einem der vorhergehenden Ansprüche zur Verwendung bei der Behandlung überempfindlicher Zähne und/oder Zahnhälse, bei der Kariesprophylaxe, bei der Behandlung beginnender Kariesläsionen, der Inhibierung von Demineralisation und/oder Zahnerosion und bei der Schmelzfluoridierung.

15. Fluoridhaltiger Lack nach einem der Ansprüche 1 bis 14, der eine Schmelzfluoridierung (ASF) nach einer Stunde Behandlungsdauer von mindestens 10 µg/cm², bevorzugt mindestens 15 µg/cm² erzeugt, gemessen an Rinderzahnschmelz.

## Claims

1. Fluoride-containing varnish for application onto the tooth surface, **characterised in that** said varnish comprises
| | |
|---|---|
| 25 - 87.5 wt % | organic solvent, |
| 2 - 50 wt % | water, |
| 5 - 50 wt % | water-insoluble film-forming agent, |
| 0.5 - 10 wt % | inorganic fluoride source in dissolved form, |
| 5 - 25 wt % | plasticiser |
in each case relative to the total weight of the varnish.

2. Fluoride-containing varnish according to claim 1 wherein the plasticiser is fatty alcohol, polyethylene glycol (PEG), polypropylene glycol (PPG), Dexpanthenol, an ester or a mixture thereof.

3. Fluoride-containing varnish according to claim 2 wherein the plasticiser is a sugar ester or an alkyl or phenyl ester of dioic or trioic acids or of hydroxydioic or hydroxytrioic acids having one or more hydroxy groups.

4. Fluoride-containing varnish according to claim 3 wherein the sugar ester is an ester of an organic acid with a mono- or disaccharide.

5. Fluoride-containing varnish according to claim 4, wherein the sugar ester is an ester of sucrose with acetic acid or iso-butanoic acid or a mixed ester of sucrose with acetic acid and iso-butanoic acid, in particular sucrose acetate iso-butyrate.

6. Fluoride-containing varnish according to one of the preceding claims, wherein the organic solvent is a water-miscible mono- or polyhydric alcohol and/or a water-miscible ketone, in particular isopropanol, acetone, quite particularly preferably ethanol.

7. Fluoride-containing varnish according to one of the preceding claims, comprising an inorganic fluoride source that has a solubility in water at 25 °C of at least 5 mol in 1 kg water, preferably at least 10 mol in 1 kg water.

8. Fluoride-containing varnish according to claim 7, comprising NH₄F, KF, RbF, CsF, NH₄HF₂, KHF₂ or a mixture thereof as the inorganic fluoride source.

9. Fluoride-containing varnish according to one of claims 1 to 8, comprising an alcohol-soluble or ketone-soluble polymer as the film-forming agent, having a solubility in water of at most 5.0 wt %.

10. Fluoride-containing varnish according to claim 9 comprising as the film-forming agent an acrylate homopolymer and methacrylate homopolymer, acrylate copolymer and methacrylate copolymer, a terpolymer of C₄-C₈ alkylacrylamide, acrylates and acrylic acid, a terpolymer of methacrylic acid and acrylates, a vinyl acetate-crotonic acid copolymer, a terpolymer of *tert-*butyl acrylate, ethyl acrylate and methacrylic acid (INCI name: acrylates copolymer) or a mixture thereof.

11. Fluoride-containing varnish according to one of the preceding claims, comprising
50 - 70 wt % ethanol,
10 - 20 wt % water,
5 - 15 wt % terpolymer of *tert*-butyl acrylate, ethyl acrylate and methacrylic acid,
10 - 20 wt % sucrose acetate isobutyrate,
1 - 2 wt % ammonium fluoride,
0.05 - 0.2 wt % flavouring substance(s) and
0.01 to 0.1 wt % sweetener.

12. Fluoride-containing varnish according to one of claims 1 to 11, additionally comprising 0.01 - 10 wt % filler and/or antibacterial agents.

13. Fluoride-containing varnish according to one of claims 1 to 12 exhibiting a viscosity of less than 5 000 mPa·s, preferably less than 1 000 mPa·s and particularly preferably less than 250 mPa·s, measured at 15.0 °C and with a shear rate of 100 s⁻¹).

14. Fluoride-containing varnish according to one of the preceding claims for use in the treatment of hypersensitive teeth and/or tooth necks, in the prophylaxis of caries, in the treatment of incipient caries lesions, the inhibition of demineralisation and/or tooth erosion and in the fluoridation of enamel.

15. Fluoride-containing varnish according to one of claims 1 to 14 which after a treatment time of one hour produces a fluoridation of enamel (ASF) of at least 10 µg/cm², preferably at least 15 µg/cm², measured on bovine tooth enamel.

## Revendications

1. Vernis fluoré à appliquer sur la surface des dents, **caractérisé en ce qu'**il contient :
- 25 à 87,5 % en poids de solvant organique,
- 2 à 50 % en poids d'eau,
- 5 à 50 % en poids d'agent filmogène insoluble dans l'eau,
- 0,5 à 10 % en poids d'une source de fluor inorganique à l'état dissous,
- et 5 à 25 % en poids d'un émollient,
par rapport à la masse totale du vernis pour chacun de ces composants.

2. Vernis fluoré conforme à la revendication 1, qui contient en tant qu'émollient un alcool gras, un polyéthylèneglycol (PEG), un polypropylèneglycol (PPG), du dexpanthénol, un ester, ou un mélange de tels corps.

3. Vernis fluoré conforme à la revendication 2, qui contient en tant qu'émollient un ester de sucre ou un ester alkylique ou phénylique d'acide dicarboxylique ou tricarboxylique ou d'acide hydroxydicarboxylique ou hydroxy-tricarboxylique comportant un ou plusieurs groupe(s) hydroxyle.

4. Vernis fluoré conforme à la revendication 3, qui contient en tant qu'ester de sucre un ester d'un acide organique et d'un monosaccharide ou d'un disaccharide.

5. Vernis fluoré conforme à la revendication 4, qui contient en tant qu'ester de sucre un ester de saccharose et d'acide acétique ou d'acide isobutyrique ou un ester mixte de saccharose et des acides acétique et isobutyrique, en particulier un acétate-isobutyrate de saccharose.

6. Vernis fluoré conforme à l'une des revendications précédentes, qui contient en tant que solvant organique un monoalcool ou polyalcool miscible à l'eau et/ou une cétone miscible à l'eau, en particulier de l'isopropanol ou de l'acétone, mais de préférence de l'éthanol.

7. Vernis fluoré conforme à l'une des revendications précédentes, qui contient une source de fluor inorganique présentant, dans de l'eau à 25 °C, une solubilité d'au moins 5 moles dans 1 kg d'eau, et de préférence, d'au moins 10 moles dans 1 kg d'eau.

8. Vernis fluoré conforme à la revendication 7, qui contient en tant que source de fluor inorganique NH₄F, KF, RbF, CsF, NH₄HF₂ ou KHF₂, ou un mélange de ces corps.

9. Vernis fluoré conforme à l'une des revendications 1 à 8, qui contient en tant qu'agent filmogène un polymère soluble dans un alcool ou dans une cétone, mais qui présente une solubilité dans l'eau d'au maximum 5,0 % en poids.

10. Vernis fluoré conforme à la revendication 9, qui contient en tant qu'agent filmogène un homopolymère d'acrylate ou de méthacrylate, un copolymère d'acrylate ou de méthacrylate, un terpolymère d'(alkyle en C₄-C₈)-acrylamide, d'acrylates et d'acide acrylique, un terpolymère d'acide méthacrylique et d'acrylates, un copolymère d'acide crotonique et d'acétate de vinyle, un terpolymère d'acrylate de tertio-butyle, d'acrylate d'éthyle et d'acide méthacrylique (nom INCI : copolymère d'acrylates), ou un mélange de tels polymères.

11. Vernis fluoré conforme à l'une des revendications précédentes, qui contient :
- 50 à 70 % en poids d'éthanol,
- 10 à 20 % en poids d'eau,
- 5 à 15 % en poids de terpolymère d'acrylate de tertio-butyle, d'acrylate d'éthyle et d'acide méthacrylique,
- 10 à 20 % en poids d'acétate-isobutyrate de saccharose,
- 1 à 2 % en poids de fluorure d'ammonium,
- 0,05 à 0,2 % en poids d'arôme(s),
- et 0,01 à 0,1 % en poids d'édulcorant.

12. Vernis fluoré conforme à l'une des revendications 1 à 11, qui contient en outre 0,01 à 10 % en poids de charge et/ou d'agents antibactériens.

13. Vernis fluoré conforme à l'une des revendications 1 à 12, qui présente une viscosité, mesurée à 15,0 °C et sous une vitesse de cisaillement de 100 s⁻¹, de moins de 5000 mPa.s, de préférence, de moins de 1000 mPa.s, et surtout, de moins de 250 mPa.s.

14. Vernis fluoré conforme à l'une des revendications précédentes, pour utilisation dans le traitement de dents et/ou collets dentaires hypersensibles, dans la prévention des caries, dans le traitement de lésions carieuses débutantes, dans l'inhibition de la déminéralisation et/ou de l'érosion dentaire, et dans la fluoration de l'émail.

15. Vernis fluoré conforme à l'une des revendications 1 à 14, qui produit, après une heure de traitement, une fluoration de l'émail (ASF, mesurée sur de l'émail dentaire de bovin) d'au moins 10 µg/cm² et de préférence d'au moins 15 µg/cm²
